Europäisches Patentamt

European Patent Office

Office européen des brevets

(19)

(11) Numéro de publication : **0 452 202 B1**

(12) **FASCICULE DE BREVET EUROPEEN**

(45) Date de publication du fascicule du brevet :
24.11.93 Bulletin 93/47

(51) Int. Cl.⁵ : **A61K 7/00,** A61K 9/16,
A61K 9/50, A61K 7/48

(21) Numéro de dépôt : **91400933.7**

(22) Date de dépôt : **05.04.91**

(54) **Composition cosmétique ou dermo-pharmaceutique aqueuse contenant en suspension des sphéroides hydratés d'une substance lipidique hydrophile.**

(30) Priorité : **05.04.90 FR 9004387**

(43) Date de publication de la demande :
**16.10.91 Bulletin 91/42**

(45) Mention de la délivrance du brevet :
**24.11.93 Bulletin 93/47**

(84) Etats contractants désignés :
**AT BE CH DE DK ES GB GR IT LI NL SE**

(56) Documents cités :
**EP-A- 0 069 423
EP-A- 0 224 457
EP-A- 0 275 358
GB-A- 2 204 792**

(73) Titulaire : **L'OREAL
14, Rue Royale
F-75008 Paris (FR)**

(72) Inventeur : **Kauffmann, Myriam
25, Boulevard des Belges
F-69006 Lyon (FR)**

(74) Mandataire : **Stalla-Bourdillon, Bernard
CABINET NONY & CIE 29, rue Cambacérès
F-75008 Paris (FR)**

EP 0 452 202 B1

Jouve, 18, rue Saint-Denis, 75001 PARIS

## Description

La présente invention a pour objet une composition cosmétique ou dermo-pharmaceutique aqueuse contenant, en suspension, de petites sphères ou sphéroïdes hydratés d'une substance lipidique hydrophile, ces sphéroïdes pouvant être chargés en au moins un composant cosmétique ou dermo-pharmaceutique liposoluble ou non liposoluble.

De nombreuses compositions topiques sont formulées sous forme aqueuse et l'on peut notamment mentionner les gels, les lotions et les émulsions, ces compositions étant des démaquillants, des toniques, des produits amincissants, des compositions anti-solaires et après soleil, des produits capillaires, des produits d'hygiène en particulier d'hygiène buccale tels que des dentifrices ainsi que certains produits de maquillage tels que des mascaras.

Ces compositions et en particulier celles à phase aqueuse continue sont tout particulièrement recherchées du fait de leur apport en eau conférant une sensation agréable de fraicheur sur la peau et la muqueuse buccale et également du fait qu'elles ne confèrent pas un aspect gras à la peau ni aux cheveux.

Ces compositions présentent toutefois certains inconvénients dans la mesure où elles peuvent provoquer un effet desséchant sur la peau, conduisant à un certain manque de confort. Par ailleurs, elles sont généralement mal supportés par les personnes présentant une peau à tendance sèche, voir même normale.

Très peu d'études ont été jusqu'ici entreprises en vue d'essayer de remédier à ces inconvénients.

En outre, il est bien connu que certains composants cosmétiques ne peuvent être introduits en quantité importante dans des gels ou des émulsions sans les déstabiliser. Il s'agit notamment des parfums et des huiles essentielles.

On a toutefois dernièrement proposé des compositions sous ferme de gels contenant des inclusions d'émulsion, mais leur préparation nécessite cependant des appareillages complexes d'injection et l'on a par ailleurs constaté qu'après prélèvement, les deux phases se mélangeaient de façon très irrégulière au niveau de la surface de la composition restant dans le conditionnement.

Après différentes études, on vient maintenant de constater que les inconvénients des compositions de l'art antérieur, à savoir l'effet desséchant, le manque de confort et la destabilisation par certains principes actifs, pouvaient être résolus par la présence, en suspension, de sphéroïdes hydratés d'une substance lipidique hydrophile.

Les sphéroïdes que l'on incorpore à l'état solide, pour une meilleure dispersion, s'hydratent et gonflent au contact de la phase aqueuse ce qui confère ainsi aux compositions de nouvelles propriétés notamment émollientes et lubrifiantes.

Les sphéroïdes hydratés de substance lipidique hydrophile permettent en effet d'empêcher l'effet de dessèchement de la peau et apportent une très agréable sensation de confort.

Par ailleurs les compositions contenant ces sphéroïdes hydratés ne nécessitent aucun appareillage particulier pour leur fabrication. Elles sont tout à fait stables vis-à-vis d'une agitation mécanique.

La présente invention a donc pour objet une composition cosmétique ou dermo-pharmaceutique aqueuse contenant, en suspension dans la phase continue, des sphéroïdes hydratés d'une substance lipidique hydrophile, lesdits sphéroïdes hydratés ayant un diamètre moyen des particules compris entre 50 et 10 000 μm.

Les sphéroïdes hydratés présents dans la composition ont une consistance crémeuse et peuvent être assimilés à des "pré-émulsions". Ils présentent la particularité, même au sein de milieux de faible viscosité (< 50 cps) de ne pas coalescer même en cas d'agitation ou de prélèvement de la composition.

La composition cosmétique ou dermo-pharmaceutique selon l'invention comporte essentiellement deux phases, une phase externe à savoir la phase continue qui peut être une émulsion, un gel ou une lotion et une phase interne constituée par les sphéroïdes hydratés que l'on peut comparer à de petits réservoirs ou globules, stériquement bien délimités, d'une pré-émulsion ou d'une crème épaisse à fort contenu lipidique formant, lors de l'application sur la peau, une émulsion cosmétiquement très agréable avec la phase externe aqueuse.

Selon l'invention et en particulier dans le cas d'un gel ou d'une lotion, la phase externe peut être de l'eau ou un mélange d'eau et d'un solvant organique hydroxylé tel que par exemple l'alcool éthylique, la glycérine, les glycols tels que le propylèneglycol ou les éthers de glycol tels que le monoéthyléther de diéthylèneglycol. Dans cette dernière forme de réalisation, le mélange aqueux contient de préférence au moins 50 % d'eau.

Lorsque la phase continue est une émulsion il convient que l'agent émulsionnant, tant d'un point de vue qualitatif que quantitatif, soit compatible avec les sphéroïdes non hydratés et hydratés, c'est-à-dire qu'il n'induise pas leur dissociation ni leur solubilisation partielle ou totale.

Par l'expression "sphéroïdes" telle qu'utilisée selon l'invention, on doit entendre de petites particules solides essentiellement sphériques alors que par l'expression "sphéroïdes hydratés" on doit entendre de petites particules crémeuses également essentiellement sphériques contenant de l'eau.

Le diamètre moyen des sphéroïdes que l'on incorpore dans la composition peut être compris dans de très larges limites, mais celui-ci doit être tel qu'après hydratation, les sphéroïdes hydratés aient un diamètre moyen des particules compris dans la gamme indiquée ci-dessus et de préférence entre 100 et 5 000 µm.

En fonction de la nature de la substance lipidique hydrophile utilisée, le gonflement des sphéroïdes par hydratation a pour effet d'augmenter voire de doubler le diamètre moyen des sphéroïdes solides introduits dans la composition.

En effet, la capacité d'hydratation des sphéroïdes est telle qu'elles peuvent absorber 1,3 à 8 fois leur poids d'eau, c'est-à-dire que leur volume est multiplié par un facteur compris entre 1,3 et 8 si l'on néglige les différences de densité. Par conséquent, leur diamètre est multiplié par la racine cubique de ce facteur, soit 1,1 à 2.

D'autre part, la nature de la phase externe aqueuse et la température peuvent moduler l'absorption d'eau par les sphéroïdes.

Le pourcentage en poids des sphéroïdes introduits dans la composition à l'état solide est bien entendu fonction de l'effet recherché, un pourcentage élevé conférant un effet adoucissant et lubrifiant plus prononcé.

Dans la pratique, ce pourcentage en poids est généralement compris entre 0,1 et 50 % mais de préférence entre 1,5 et 10 % en poids.

Les substances lipidiques hydrophiles pour la formation des sphéroïdes doivent être solides à température ambiante, c'est-à-dire présenter un point de fusion supérieur à 20 °C. Le point de fusion maximum n'est pas critique mais l'on préfère utiliser des substances lipidiques hydrophiles ayant un point de fusion inférieur à 80 °C.

Parmi les substances lipidiques solides hydrophiles particulièrement préférées pour la préparation des sphéroïdes, on peut notamment mentionner :

(1) Les alcools gras en $C_{12}$-$C_{24}$ ayant un point de fusion compris entre 20 et 80 °C et ayant un indice d'hydroxyle (IOH) compris entre 100 et 300.

Parmi ces alcools gras ceux particulièrement préférés sont : l'alcool myristique, l'alcool cétylique et l'alcool stéarylique.

(2) Les esters partiels d'acides gras en $C_{12}$-$C_{24}$ avec des polyols ou oligomères de polyols tels que l'éthylène glycol, le propylène glycol, le glycérol, des sucres en $C_3$-$C_6$ linéaires, ramifiés ou cycliques ou le diéthylène glycol, les polyéthylène glycols, les polyglycérols et le saccharose.

Ces esters partiels doivent avoir un point de fusion compris entre 20 et 80 °C et un indice d'hydroxyle (IOH) compris entre 50 et 500 ou une HLB (Hydrophile-Lipophile Balance) comprise entre 1 et 13.

Parmi ces esters partiels on peut notamment citer: le monodipalmitostéarate de glycérol tel que le "GELEOL" vendu par la Société GATTEFOSSE, le nonopalmitate de Sorbitan tel que l' "ARLACEL 40" vendu par la Société ICI, le monostéarate de diéthylène glycol tel que le "TEIGIN D" vendu par la Société GOLDSCHMIDT ou le béhénate de glycérol tel que le "COMPRITOL" vendu par la Société GATTEFOSSE.

(3) Les dérivés oxyéthylénés de corps gras tels que ceux d'alcools gras en $C_{12}$-$C_{24}$ et de leurs esters, d'acide gras en $C_{12}$-$C_{24}$ et de leurs esters, d'amines et amides gras en $C_{12}$-$C_{24}$, et de cires, de lanolines et d'huiles hydrogénées et leurs mélanges, ces corps gras étant oxyéthylénés à l'aide de 2 à 50 moles d'oxyde d'éthylène (OE) par mole de corps gras.

Ces dérivés oxyéthylénés de corps gras doivent avoir un point de fusion de 20 à 80 °C et une HLB comprise entre 1 et 13.

Parmi ceux-ci on peut notamment mentionner :
l'alcool stéarylique oxyéthyléné à 2 moles d'OE, tel que le "BRIJ 72" vendu par la Société I.C.I., le distéarate de polyéthylèneglycol à 8 moles d'OE, tel que le "LIPOPEG 4 DS" vendu par la Société LIPO ou la cire d'abeilles hydrophile telle que l' "APIFIL" vendue par la Société GATTEFOSSE.

(4) Les produits issus de la réaction d'alcoolyse entre les triglycérides et les polyoxyéthylèneglycols, ayant un point de fusion compris entre 20 et 80 °C, et un indice d'hydroxyle compris entre 50 et 500 ou une HLB comprise entre 1 et 13.

Il s'agit en particulier des glycérides lauropalmitostéariques polyoxyéthylénés glycolysés (huiles de palme/palmiste hydrogénées interestérifiées), tels que les "LABRAFILS M2130 BS, M2130 CS et WL2514 CS", vendus par la Société GATTEFOSSE.

(5) Les phospholipides et sphingolipides et leurs dérivés hydrogénés de point de fusion supérieur à 20 °C, ayant un indice d'hydroxyle compris entre 50 et 500 ou une HLB comprise entre 1 et 13.

(6) Les silicones amphiphiles tels que les diméthicone copolyols, les polyalkyl diméthicone copolyols, ou leurs dérivés portant des esters à longue chaîne, comportant 2 à 50 moles d'OE par mole de produit, et ayant un point de fusion compris entre 20 et 80 °C.

Les substances lipidiques hydrophiles telles qu'énumérées ci-dessus peuvent être utilisées seules ou éventuellement sous forme d'un mélange.

Il est également possible d'utiliser des mélanges de substances lipidiques, chacune d'elles pouvant avoir une ou plusieurs caractéristiques (point de fusion, indice d'hydroxyle, HLB) hors des limites précédemment définies, mais dont l'association possède les caractéristiques physico-chimiques énoncées.

Il est en outre possible pour ajuster la consistance ou la viscosité des sphéroïdes, d'introduire dans le mélange, des argiles modifiées ou leur dispersion huileuse, des silices, des savons métalliques ou tout autre structurant des corps gras.

Selon une forme de réalisation préférée de l'invention, les sphéroïdes de substance lipidique hydrophile contiennent dissous ou dispersés dans leur matrice, des composants cosmétiques ou dermo-pharmaceutiques.

Selon cette forme de réalisation, la substance lipidique hydrophile des sphéroïdes sert de support et de véhicule à des composants tels que des parfums, des huiles essentielles ou leurs constituants, des pigments, des charges, des colorants, des vitamines, des enzymes et diverses autres substances actives au sens de la cosmétique qui peuvent être présents à raison de 0,01 à 70 % et de préférence de 1 à 40 % en poids par rapport au poids des sphéroïdes.

Il va de soi que lorsque l'on incorpore ou charge de tels composants cosmétiques ou dermo-pharmaceutiques dans les sphéroïdes de substance lipidique hydrophile, il convient de choisir une substance lipidique hydrophile appropriée de telle sorte qu'après granulation, les sphéroïdes se présentent toujours sous forme solide avant leur incorporation dans la composition, et conservent toujours leur capacité d'absorption d'eau.

L'incorporation de parfums et d'huiles essentielles dans les sphéroïdes est particulièrement appropriée car il est bien connu que ceux-ci sont des facteurs de déstabilisation.

Cette forme de réalisation par incorporation de sphéroïdes chargées en parfum, en huiles essentielles ou leurs constituants, permet de rendre possible la formulation de produits, à phase externe aqueuse, particulièrement riches en ces composés, ce qui jusqu'à présent ne pouvait être obtenu.

Parmi les composants cosmétiques ou dermo-pharmaceutiques liposolubles qui peuvent être incorporés dans les sphéroïdes, on peut mentionner les vitrines ou pro-vitamines plus ou moins liposolubles, telles que les vitamines A et E et leurs esters, les esters de la vitamine C, les caroténoïdes, les substances anti-radicalaires, antiseptiques, anti-acnéiques ou anti-pelliculaires, les filtres U.V., les kératolytiques tels que l'acide salicylique ou ses dérivés ou ses sels, les molécules agissant sur la pigmentation, sur l'inflammation telles que les esters de zinc ou de cuivre et d'acides gras, les esters gras émollients, les huiles d'origine minérale, animale, végétale ou synthétique, les substituts ou composants du sébum tels que le squalène, le squalane et les céramides, le cholestérol, les extraits biologiques ou les colorants du cheveu, etc...

Il est également possible d'incorporer dans ces sphères des charges adoucissantes ou lubrifiantes pour la peau telles que du talc, des microbilles de polyamide, ou au contraire des particules destinées à produire un frottement ou une abrasion de la peau ou des dents, telles que des poudres de polyéthylène ou d'autres matières plastiques, des débris végétaux, cellulosiques ou ligniques, ou des particules minérales telles que des poudres de silice.

Les sphéroïdes peuvent également contenir un faible pourcentage d'autres composants habituels des phases lipidiques cosmétiques ou dermo-pharmaceutiques tels que des antioxydants, des conservateurs et des colorants.

Enfin ces sphéroïdes peuvent contenir eux-mêmes d'autres sphères, capsules ou systèmes vecteurs moléculaires dont la taille peut aller de quelques angströms jusqu'à quelques centaines de microns ou tout autre système microcapsulaire ou micromatriciel contenant lui-même des principes actifs. La formule globale est alors pluricompartimentée, chaque compartiment étant immobilisé.

Selon une forme de réalisation préférée de l'invention les compositions contiennent en suspension des sphéroïdes hydratés chargés à l'aide de composants cosmétiques ou dermo-pharmaceutiques différents ce qui présente un intérêt particulier dans le cas de composants incompatibles entre eux tels que des parfums ou des huiles insaturées et des métaux ou des oxydes métalliques.

La présente invention a également pour objet un procédé de préparation des compositions telles que définies ci-dessus, ce procédé consistant à incorporer, sous agitation, les sphéroïdes solides, chargés ou non chargés, dans la phase continue de façon à former une suspension et à la soumettre ensuite à une étape dite de "maturation" pendant une durée déterminée et à une température contrôlée, le traitement étant fonction de la nature de la substance lipidique hydrophile utilisée pour former les sphéroïdes et de la nature de la phase aqueuse externe.

Cette dernière étape dite de "maturation" provoque l'hydratation des sphéroïdes et a pour conséquence leur gonflement et leur ramollissement.

Le phénomène qui est visible macroscopiquement, s'accompagne d'une augmentation du diamètre et d'une modification éventuelle de la couleur et de l'opacité des sphéroïdes notamment lorsqu'ils sont chargés en une substance colorante ou en pigment(s).

L'étape de maturation est généralement effectuée à une température comprise entre 15 et 80 °C et pendant une durée de 1 heure à 15 jours.

Les sphéroïdes solides peuvent être préparés par toutes méthodes conventionnelles de granulation ou de sphéronisation à chaud ou à froid.

Par exemple, par solidification par le froid de gouttelettes de la substance lipidique hydrophile fondue, maintenue en mouvement dans un liquide non solvant tel que l'eau, ou par refroidissement par un gaz froid de gouttelettes de la substance lipidique hydrophile pulvérisée, projetée sur un disque rotatif, ou extrudée, ou par granulation à chaud autour d'un noyau solide dans une turbine ou par un lit d'air fluidisé, ou dans un mélangeur planétaire, par passage à travers une filière ou une grille vibrante, par moulage, coulage ou injection à chaud ou sous pression dans des moules, ou par découpage ou division d'une masse lipidique solide.

Les méthodes de préparation à froid, ou requérant peu d'échauffement, sont généralement préférées lorsque les composants cosmétiques ou dermo-pharmaceutiques inclus dans la substance lipidique hydrophile sont fragiles vis-à-vis de la température ou de l'oxydation. Le refroidissement des gouttelettes fondues est obtenu de préférence par un gaz plutôt que par l'eau.

Selon le procédé choisi et les paramètres de fabrication, les sphéroïdes solides obtenus sont parfaitement calibrés ou de distribution de taille ou de diamètre plus ou moins étalé.

Les sphéroïdes solides peuvent être manipulés industriellement sans précaution particulière. Leur introduction dans la phase externe peut s'effectuer directement dans le conditionnement définitif, dans lequel l'étape de maturation aura lieu.

Les compositions selon l'invention sont comme indiqué précédemment des gels, des lotions ou des émulsions du type eau-dans-l'huile ou huile-dans-l'eau mais de préférence des gels.

Les gels dans lesquels sont incorporés les sphéroïdes sont obtenus à l'aide d'un agent gélifiant, généralement présent à une concentration comprise entre 0,02 et 70 % en poids par rapport au poids total du gel. (y compris les sphéroïdes hydratés chargés ou non chargés).

Parmi les agents gélifiants particulièrement appropriés pour la formation des gels selon l'invention on peut notamment mentionner les polymères carboxyvinyliques tels que les CARBOPOLS, neutralisés par une base minérale ou organique (soude ou triéthanolamine), les gélifiants polysaccharidiques tels que les alginates, les gommes de xanthane, les dérivés de la cellulose, ou encore la gélatine ou les gélifiants minéraux tels que les bentones ou les silices modifiées.

Ces gels peuvent également contenir dans la phase aqueuse continue divers adjuvants cosmétiquement acceptables hydrosolubles et en particulier des colorants, des agents hydratants, des extraits biologiques, des vitamines ou des acides aminés.

On va maintenant donner à titre d'illustration plusieurs exemples de préparation des sphéroïdes solides chargés ou non chargés ainsi que plusieurs exemples de préparations des gels selon l'invention.

PROCEDE DE PREPARATION DES SPHEROÏDES SOLIDES

L'ensemble substance(s) lipidique(s) hydrophile(s) + éventuellement actif(s) est préparé à chaud. Le ou les composants solides de la substance lipidique hydrophile sont fondus à une température supérieure de 2 à 3 °C à celle de la substance lipidique hydrophile du plus haut point de fusion. Puis on ajoute les autres composants, en commençant par les moins fragiles.

Le mélange est maintenu à une température supérieure de 2 à 3 °C à celle de la zone de solidification. Les composants fragiles ou volatils sont ajoutés en dernier. Les solides sont ajoutés à la fin dans le mélange fondu, et maintenus sous agitation. Ils peuvent être éventuellement "empatés" par une fraction de la substance lipidique hydrophile fondue avant leur incorporation à la totalité du mélange.

Lorsque le mélange est homogène, la masse fondue est pulvérisée dans la partie supérieure colonne verticale d'air froid. Les goutelettes de lipides fondus tombent par gravitation tout en refroidissant.

Les gouttelettes solidifiées sont récupérées à la base de la colonne.

Selon un autre procédé, il est également possible de couler la masse lipidique fondue dans une phase aqueuse portée à la même température et maintenue sous agitation circulaire : le mélange lipidique se disperse sous forme de gouttelettes sphériques. Un agent gélifiant est alors ajouté dans le milieu, qui est refroidi sous agitation. Dans ce cas, la préparation des sphéroïdes est réalisée simultanément à leur incorporation dans le milieu externe final.

Selon l'un de ces modes opératoires, on a préparé des sphéroïdes solides non chargés et chargés en diverses substances, selon les exemples suivants :

1) Exemple de sphéroïdes non chargés :

Alcool stéarylique à 2 moles d'OE vendu par la Société I.C.I. sous la
dénomination de "BRIJ 72"................................. 100    g

Taille moyenne des particules : 1000 µm

2) Exemple de sphéroïdes chargés en vaseline :

Huile de vaseline....................................... 10    g
Vaseline................................................ 19    g
Monodipalmitostéarate de glycérol....................... 60    g
Glycéride oléique interesterifié vendu sous la dénomination
de "LABRAFIL M2735 CS" par la Société GATTEFOSSE ......... 10    g
Parfum.................................................. 1    g

Taille moyenne des particules : 1 500 µm (couleur ivoire)

3) Exemple de sphéroïdes chargés en pigments :

Exemple 3-A

Oxyde de fer jaune ..................................... 3    g
Oxyde de fer rouge...................................... 1,5  g
Oxyde de fer noir....................................... 0,5  g
Dioxyde de titane....................................... 15    g
Huile de palme/palmiste hydrogénée interestérifiée......... 10    g
Cire d'abeilles hydrophile................................ 60    g
Macération de carottes dans l'huile de soja................ 10    g

Taille moyenne des particules : 500 µm (couleur brune)

Exemple 3-B :

Dioxyde de titane....................................... 20    g
Huile de palme/palmiste hydrogénées interestérifiées....... 10    g
Cire d'abeilles hydrophile................................ 60    g
Macération de carottes dans l'huile de soja................ 10    g

Taille moyenne des particules : 500 µm (couleur blanche)

Exemple 3-C :

| | |
|---|---|
| Monodipalmitostéarate de glycérol............................. | 68,75g |
| Fraction liquide de beurre de karité......................... | 15  g |
| Miglyol gel................................................... | 8  g |
| Oxyde de fer ocre............................................. | 1,4 g |
| Oxyde de fer brun rouge....................................... | 0,8 g |
| Oxyde de fer noir............................................. | 0,2 g |
| Dioxyde de titane............................................. | 5,6 g |
| BHT........................................................... | 0,05g |
| Conservateur.................................................. | 0,2 g |

Taille moyenne des particules : comprise entre 600 et 800 μm (couleur pourpre)

Exemple 3-D :

| | |
|---|---|
| Monodipalmitostéarate de glycérol............................ | 68,75g |
| Fraction liquide de beurre de karité........................ | 15  g |
| Miglyol gel.................................................. | 8  g |
| Dioxyde de chrome ........................................... | 0,5 g |
| Dioxyde de titane........................................... | 7,5 g |
| BHT......................................................... | 0,05g |
| Conservateur................................................ | 0,2 g |

Taille moyenne des particules : comprise entre 400 et 500 μm (couleur vert clair)

4) Exemple de sphéroïdes chargés en parfum :

| | |
|---|---|
| Concentré de parfum.......................................... | 30  g |
| Monodipalmitostéarate de glycérol............................ | 54  g |
| Monodipalmitostéarate de polyéthylèneglycol.................. | 15,75g |
| Butylhydroxytoluène.......................................... | 0,05g |
| Conservateur................................................. | 0,2 g |

Taille moyenne des particules : 1 000 μm (couleur jaune pâle)

7

5) Exemple de sphéroïdes chargés en insaponifiables de soja :

```
Extrait huileux d'insaponifiables de soja..................   40    g
Monodipalmitostéarate de glycérol..........................   55    g
Alcool myristique..........................................   1,748g
Palmitate de vitamine A....................................    3    g
DC Green 6.................................................   0,002g
Butylhydroxytoluène........................................   0,05 g
Conservateur...............................................   0,2  g
```

Taille moyenne des particules : comprise entre 500 et 1 200 µm (couleur verte)

6) Exemple de sphéroïdes chargés en agent cicatrisant :

```
Monostéarate de Sorbitan...................................   88    g
Extrait huileux de calendula...............................   10    g
∠ -Bisabolol...............................................    1    g
Nacre bleue................................................    1    g
```

Taille moyenne des particules : 500 µm, sphéroïdes dures de couleur bleue.

## EXEMPLES DE PREPARATION DES COMPOSITIONS

## EXEMPLE A

Préparation d'un gel démaquillant tonique

Dans un flacon approprié on conditionne 80 g d'un gel ayant la composition suivante :

```
"CARBOPOL 940" de la Société GOODRICH......................   0,15 g
Hydroxyde de sodium qsp    pH : 6,5
Glycérine..................................................    3    g
Eau d'hamamélis ...........................................   20    g
Eau de rose ...............................................   20    g
FD et C Blue I.............................................   0,002g
Conservateur ..............................................   0,02 g
Eau ........q.s.p. ........................................  100    g
```

A ce gel on incorpore sous agitation 20 g de sphéroïdes solides chargés en vaseline obtenus selon l'exemple 2.

Le flacon est alors soumis à l'étape de maturation en le plaçant dans une enceinte à 45 °C pendant 3 jours.

A la fin de cette période de maturation on obtient un gel fluide transparent bleuté contenant des sphéroïdes hydratés de couleur blanche ayant un diamètre moyen de 2 500 µm.

Ce gel constitue un excellent démaquillant qui ne nécessite pas de rinçage à l'eau ou avec un tonique. Il ne laisse pas sur la peau de résidus gras ni ne provoque de sensation d'inconfort due à un effet desséchant.

## EXEMPLE B

Préparation d'un fond de teint

Dans un flacon approprié on conditionne 91 g de gel ayant la composition suivante :

```
"CARBOPOL 940" de la Société GOODRICH......................    0,25g
Triéthanolamine q.s.p    pH : 6,5
Glycérine.................................................    4    g
Conservateur .............................................    0,2 g
Eau........q.s.p..........................................   100    g
```

A ce gel on incorpore sous agitation 3 g de sphéroïdes solides chargés en pigments obtenus selon l'exemple 3-A, et 3 g de sphéroïdes solides chargés en pigments obtenus selon l'exemple 3-B.

Le flacon ainsi-conditionné est alors soumis à l'étape de maturation en le plaçant dans une enceinte appropriée à une température de 37 °C pendant 4 jours.

A la fin de cette étape de maturation on obtient un gel transparent contenant en dispersion les sphéroïdes hydratés de différentes couleurs (brun clair, ocre et blanche) ayant un diamètre d'environ 1 000 µm.

Par application sur le visage ce gel s'homogénéise rapidement sous les doigts. L'excipient est rapidement absorbé et les pigments réalisent un maquillage mat et homogène particulièrement adapté aux peaux grasses.

## EXEMPLE C

Préparation d'un gel parfumé

Dans un flacon approprié on conditionne 95 g d'un gel ayant la composition suivante :

```
"CARBOPOL 940" de la Société GOODRICH......................    0,3 g
Triéthanolamine q.s.p. pH : 6,5
Méthylparaben sodé........................................    0,2 g
Glycérine.................................................    4    g
Eau distillée........ q.s.p ..............................   100    g
```

On incorpore alors dans le flacon 5 g de sphéroïdes solides chargés en parfum obtenus selon l'exemple 4 à l'aide d'un mélangeur planétaire.

Après maturation, pendant une semaine dans une enceinte appropriée à 20 °C, on obtient un gel transparent contenant en inclusion des sphéroïdes hydratés de couleur jaune très pâle très fondants ayant un diamètre moyen de 2 000 µm qui s'émulsionnent facilement sur la peau avec le gel.

Ce produit parfumé peut être appliqué sur tout le corps.

## EXEMPLE D

Préparation d'un gel pour soin

Dans un flacon approprié on conditionne 96 g d'un gel ayant la composition suivante :

```
"CARBOPOL 940" de la Société GOODRICH ....................    0,35 g

Méthylparaben sodé...q.s.

Eau distillée........q.s.p.................................   100    g
```

A ce pré-gel on incorpore 4 g de sphéroïdes solides chargés en insaponifiables de soja obtenus selon l'exemple 5 à l'aide d'un mélangeur planétaire, puis toujours sous agitation, le pré-gel est amené à un pH voisin de sa neutralité par adjonction de 0,35 g de triéthanolamine. L'agitation est arrêtée lorsque le gel atteint une viscosité stable.

Après maturation pendant 4 jours à 37 °C dans une enceinte appropriée, on obtient un gel transparent contenant en inclusion des sphéroïdes hydratés ayant un diamètre compris entre 700 et 2 000 µm de couleur vert très pâle qui s'émulsionnent immédiatement sur la peau lors de l'application.

EXEMPLE E : Préparation d'une émulsion eau-dans-l'huile :

Dans un flacon approprié on conditionne 96 g d'une émulsion ayant la composition suivante :

```
Huile de vaseline.......................................    6   g

Vaseline................................................    6   g

Paraffine...............................................    3   g

Glycéride partiel de l'acide isostéarique...............    6   g

Association de triglycérides capryliques/capriques et de

bentone.................................................   15   g

Huile de silicone volatile (pentamère)..................    6   g

Glycérine...............................................    5   g

Sulfate de magnésium à 7 molécules d'eau................    1   g

Conservateur..............q.s.

Eau distillée q.s.p.  ..................................  100   g
```

Celle-ci est préparée selon les techniques habituellement connues et se présente sous forme d'une crème blanche.

On y incorpore 4 g des sphéroïdes solides chargés en agent cicatrisant obtenus selon l'exemple 6.

Après une semaine de maturation, les sphéroïdes ont gonflé et se sont transformés en sphéroïdes hydratés de diamètre d'environ 800 µm de couleur bleutée et de consistance fondante, qui restent parfaitement individualisés dans la crème.

Leur pouvoir cicatrisant s'ajoute à l'effet adoucissant et protecteur de l'émulsion eau-dans-l'huile. Ce produit est particulièrement recommandé pour les peaux sèches et irritées.

EXEMPLE F : Préparation d'une émulsion huile-dans-l'eau

Dans un flacon approprié on conditionne 95 g d'une émulsion ayant la composition suivante :

```
       Huile de ricin................................................      8     g

       Huile de jojoba...............................................      5     g

       Monodipalmitostéarate de glycérol.........................      6     g

       "CARBOPOL 940" de la Société GOODRICH......................    0,2    g

       Soude.........................................................    0,1   g

       Conservateur..........  q.s.

       Antioxydant............ q.s.

       Eau distillée q.s.p...........................................    100    g
```

Celle-ci est préparée selon les techniques habituellement connues et se présente sous forme d'une crème blanche.

On y incorpore sous agitation 5 g de sphéroïdes solides chargés en insaponifiables de soja obtenus selon l'exemple 5.

L'ensemble est soumis à une maturation de 48 H à 37 °C.

Le produit résultant est une émulsion huile-dans-l'eau de texture légère, contenant des sphéroïdes hydratés vert pâle de diamètre compris entre 700 et 2 000 μm, bien individualisés, mais qui s'émulsionnent avec la crème sur la peau lors de l'application.

Les sphéroïdes hydratés permettent un apport supplémentaire d'actifs par rapport à la crème.

EXEMPLE G : Préparation d'une lotion

A 85 g d'une lotion ayant la composition suivante :

```
       Eau de rose............................................    10    g

       Eau de bleuet..........................................    10    g

       Glycérine..............................................     5    g

       Conservateur...........q.s.

       Colorant...............q.s.

       Eau distillée..........q.s.p......................    100    g
```

on incorpore 15 g de sphéroïdes solides chargés en insaponifiables de soja obtenus selon l'exemple 5.

L'ensemble est soumis à une maturation de 48 H à 37 °C.

Le produit résultant est une lotion qui contient en suspension des sphéroïdes hydratés vert pâle de diamètre compris entre 700 et 2 000 μm, bien individualisés, non coalescents, et s'émulsionnant facilement avec la lotion lors de l'application sur la peau.

Ces sphéroïdes hydratés permettent d'enrichir la lotion en actifs lipidiques et améliorent son confort.

EXEMPLE H : Préparation d'un gel de maquillage

Dans 95,5 g d'un gel ayant la composition suivante :

"CARBOPOL 940" de la Société GOODRICH.................     0,3 g

Méthylparaben sodé...................................     0,2 g

Glycérine............................................     5   g

Filtre solaire......................................     0,1 g

Eau distillée..........q.s.p.........................     100   g

on incorpore sous agitation 3,5 g de sphéroïdes solides chargés en pigments obtenus selon l'exemple 3-C et 1 g de sphéroïdes solides chargés en pigments obtenus selon l'exemple 3-D.

On conditionne alors le gel ayant en suspension les sphéroïdes solides dans un tube souple transparent. Le tube est alors soumis à une maturation pendant 5 jours a 37 °C.

On obtient un gel transparent contenant en inclusion de grosses sphères crémeuses couleur brun rose, de diamètre d'environ 1 000 à 1 500 μm, riches en pigments et de petites spheres vert pâle de même consistance ayant un diamètre d'environ 700 à 900 μm à fonction correctrice de teint.

Une pression sur le tube permet d'éjecter le gel sans nuire a l'intégrité des sphères. Lorsque le produit est appliqué sur la peau, l'ensemble des pigments s'émulsionne et s'homogénéise sous les doigts en une crème légère, et réalise un maquillage du teint à consistance agréable.

## Revendications

1. Composition cosmétique ou dermo-pharmaceutique aqueuse, caractérisée par le fait qu'elle contient, en suspension dans la phase continue, des sphéroïdes hydratés d'une substance lipidique hydrophile, lesdits sphéroïdes hydratés ayant un diamètre moyen des particules compris entre 50 et 10 000 μm.

2. Composition selon la revendication 1, caractérisée par le fait que la phase externe est de l'eau ou un mélange d'eau et d'un solvant organique hydroxylé, ledit mélange contenant au moins 50 % d'eau.

3. Composition selon la revendication 1, caractérisée par le fait que la phase externe est un gel, l'agent gélifiant étant présent en une proportion comprise entre 0,02 et 70 % en poids par rapport au poids total de la composition.

4. Composition selon la revendication 1, caractérisée par le fait que la phase externe est une émulsion du type huile-dans-l'eau ou eau-dans-l'huile.

5. Composition selon la revendication 1, caractérisée par le fait que le diamètre moyen des particules est de préférence compris entre 100 et 5 000 μm.

6. Composition selon l'une quelconque des revendications 1 à 5, caractérisée par le fait que la substance lipidique hydrophile est un alcool gras en $C_{12}$-$C_{24}$ ayant un point de fusion compris entre 20 et 80 °C et ayant un indice d'hydroxyle compris entre 100 et 300.

7. Composition selon l'une quelconque des revendications 1 à 5, caractérisée par le fait que la substance lipidique hydrophile est un ester partiel d'acides gras en $C_{12}$-$C_{24}$ avec des polyols ou oligomères de polyols, ayant un point de fusion compris entre 20 et 80 °C et un indice d'hydroxyle compris entre 50 et 500 ou ayant une HLB comprise entre 1 et 13.

8. Composition selon l'une quelconque des revendications 1 à 5, caractérisée par le fait que la substance lipidique hydrophile est un dérivé oxyéthyléné de corps gras à l'aide de 2 à 50 moles d'oxyde d'éthylène, ayant un point de fusion compris entre 20 et 80 °c et ayant une HLB comprise entre 1 et 13.

9. Composition selon l'une quelconque des revendications 1 à 5, caractérisée par le fait que la substance lipidique hydrophile est un produit issu de la réaction d'alcoolyse entre les triglycérides naturels et les polyoxyéthylèneglycols, ayant un point de fusion compris entre 20 et 80 °C, un indice d'hydroxyle compris entre 50 et 500 ou une HLB comprise entre 1 et 13.

10. Composition selon l'une quelconque des revendications 1 à 5, caractérisée par le fait que la substance lipidique hydrophile est un phospholipide ou un sphingolipide ou un de leurs dérivés ayant un point de fusion supérieur à 20 °C et ayant un indice d'hydroxyle compris entre 50 et 500 ou une HLB comprise entre 1 et 13.

11. Composition selon l'une quelconque des revendications 1 à 4, caractérisée par le fait que la substance lipidique hydrophile est une silicone amphiphile ayant un point de fusion compris entre 20 et 80 °C et comportant de 2 à 50 moles d'oxyde d'éthylène par mole de produit.

12. Composition selon l'une quelconque des revendications précédentes, caractérisée par le fait que les sphéroïdes hydratés sont chargés en au moins un composant cosmétique ou dermo-pharmaceutique liposoluble ou non liposoluble.

13. Composition selon la revendication 12, caractérisée par le fait que les sphéroides hydratés sont chargés en un parfum, une huile essentielle, un pigment, une charge, une substance abrasive, un colorant et/ou une substance active cosmétique ou dermo-pharmaceutique.

14. Composition selon l'une quelconque des revendications 12 et 13, caractérisée par le fait que les sphéroides hydratés sont chargés en au moins un ingrédient cosmétique à raison de 0,01 à 70 % et de préférence de 1 à 40 % en poids par rapport au poids des sphéroïdes.

15. Composition selon l'une quelconque des revendications précédentes, caractérisée par le fait que la phase continue contient au moins un adjuvant cosmétique ou dermo-pharmaceutique hydrosoluble pris dans le groupe constitué par : un colorant, un agent hydratant, un extrait biologique, une vitamine et un acide aminé.

16. Procédé de préparation des compositions selon l'une quelconque des revendications 1 à 15, caractérisé par le fait qu'il consiste à incorporer dans la phase continue, des sphéroides d'une substance lipidique hydrophile, à l'état solide, lesdits sphéroides étant chargés ou non chargés, de façon à former une suspension et à soumettre la composition à une étape de maturation de façon à provoquer l'hydratation et le gonflement des sphéroïdes.

17. Procédé selon la revendication 16, caractérisé par le fait que l'on incorpore dans la composition les sphéroïdes en une proportion comprise entre 0,1 et 50% en poids et de préférence entre 1,5 et 10% par rapport au poids total de la composition.

18. Procédé selon l'une quelconque des revendications 16 et 17, caractérisé par le fait que l'étape de maturation est effectuée à une température comprise entre 15 °C et 80 °C pendant une durée d'une heure à 15 jours.


**Patentansprüche**

1. Kosmetische oder dermopharmazeutische wäßrige Zubereitung, dadurch gekennzeichnet, daß sie in Suspension in der kontinuierlichen Phase hydratisierte Sphäroide einer hydrophilen Lipidsubstanz enthält, wobei die hydratisierten Sphäroide einen mittleren Durchmesser der Teilchen zwischen 50 und 10.000 $\mu$m aufweisen.

2. Zubereitung nach Anspruch 1, dadurch gekennzeichnet. daß die externe Phase aus Wasser oder einer Mischung von Wasser mit einem organischen, Hydroxylgruppen-haltigen Lösungsmittel besteht, wobei die Mischung wenigstens 50 % Wasser enthält.

3. Zubereitung nach Anspruch 1, dadurch gekennzeichnet, daß die externe Phase ein Gel ist, wobei das gelbildende Mittel in einem Mengenanteil zwischen 0,02 und 70 Gew.-% zugegen ist, bezogen auf das Oesamtgewicht der Zubereitung.

4. Zubereitung nach Anspruch 1, dadurch gekennzeichnet, daß die externe Phase eine Emulsion des Typs Öl-in-Wasser oder Wasser-in-Öl ist.

5. Zubereitung nach Anspruch 1, dadurch gekennzeichnet, daß der mittlere Teilchendurchmesser vorzugsweise zwischen 100 und 5.000 $\mu$m liegt.

6. Zubereitung nach einem der Ansprüche 1 bis 5, dadurch gekennzeichnet, daß die hydrophile Lipidsubstanz ein Fettalkohol mit 12 bis 24 Kohlenstoffatomen ist, der einen Schmelzpunkt zwischen 20 und 80 °C aufweist und eine Hydroxylzahl zwischen 100 und 300 aufweist.

7. Zubereitung nach einem der Ansprüche 1 bis 5, dadurch gekennzeichnet, daß die hydrophile Lipidsubstanz ein Partialester von Fettsäuren mit 12 bis 24 Kohlenstoffatomen mit Polyolen oder Oligomeren von Polyolen ist, der einen Schmelzpunkt zwischen 20 und 80 °C und eine Hydroxylzahl zwischen 50 und 500 aufweist oder einen HLB-Wert zwischen 1 und 13 hat.

8. Zubereitung nach einem der Ansprucbe 1 bis 5, dadurch gekennzeichnet, daß die hydrophile Lipidsubstanz ein ethoxyliertes Derivat von einem Fettstoff mit 2 bis 50 Mol Ethylenoxid ist, das einen Schmelzpunkt zwischen 20 und 80 °C aufweist und einen HLB-Wert zwischen 1 und 13 hat.

9. Zubereitung nach einem der Ansprüche 1 bis 5, dadurch gekennzeichnet, daß die hydrophile Lipidsubstanz ein Produkt ist, das bei der Alkoholyse-Reaktion zwischen natürlichen Triglyceriden und Polyoxyethylenglykolen entsteht und einen Schmelzpunkt zwischen 20 und 80 °C, eine Hydroxylzahl zwischen 50 und 500 oder einen HLB-Wert zwischen 1 und 13 hat.

10. Zubereitung nach einem der Ansprüche 1 bis 5, dadurch gekennzeichnet, daß die hydrophile Lipidsustanz ein Phospholipid oder ein Sphingolipid oder eines ihrer Derivate ist, das einen Schmelzpunkt oberhalb von 20 °C aufweist und eine Hydroxylzahl zwischen 50 und 500 oder einen HLB-Wert zwischen 1 und 13 hat.

11. Zubereitung nach einem der Ansprüche 1 bis 4, dadurch gekennzeichnet, daß die hydrophile Lipidsubstanz ein amphiphiles Silicon ist, das einen Schmelzpunkt zwischen 20 und 80 °C aufweist und das 20 bis 50 Mol Ethylenoxid pro Mol des Produktes umfaßt.

12. Zubereitung nach einem der vorangehenden Ansprüche, dadurch gekennzeichnet, daß die hydratisierten Sphäroide mit wenigstens einer kosmetischen oder dermopharmazeutischen fettlöslichen oder nicht fettlöslichen Verbindung beladen sind.

13. Zubereitung nach Anspruch 12, dadurch gekennzeichnet, daß die hydratisierten Sphäroide mit einem Duftstoff, einem essentiellen Öl, einem Pigment, einem Füllstoff, einer schleifend wirkenden Substanz, einem Färbemittel und/oder einer kosmetischen oder dermopharmazeutischen Aktivsubstanz beladen sind.

14. Zubereitung nach einem der Ansprüche 12 und 13, dadurch gekennzeichnet, daß die hydratisierten Sphäroide mit wenigstens einer kosmetischen Substanz in einer Menge von 0,01 bis 70 Gew.-%, vorzugsweise von 1 bis 40 Gew.-%, bezogen auf das Gewicht der Sphäroide, beladen sind.

15. Zubereitung nach einem der vorangehenden Ansprüche, dadurch gekennzeichnet, daß die kontinuierliche Phase wenigstens einen in Wasser loslichen kosmetischen oder dermopharmazeutischen Hilfsstoff aus der aus einem Färbemittel, einem Hydratationsmittel, einem biologischen Extrakt, einem Vitamin und einer Aminosäure bestehenden Gruppe enthält.

16. Verfahren zur Herstellung der Zubereitungen nach einem der Ansprüche 1 bis 15, dadurch gekennzeichnet, daß es daraus besteht, daß man in die kontinuierliche Phase Sphäroide einer hydrophilen Lipidsubstanz in festem Zustand einarbeitet, wobei die Sphäroide beladen sind oder nicht beladen sind, und so eine Suspension bildet und die Zubereitung einem Schritt der Reifimg unterwirft, um die Hydratation und Quellung der Sphäroide zu bewirken.

17. Verfahren nach Anspruch 16, dadurch gekennzeichnet, daß man in die Zubereitung die Sphäroide in einem Mengenanteil zwischen 0,1 und 50 Gew.-%, vorzugsweise zwischen 1,5 und 10 Gew.- % einarbeitet, bezogen auf das Gesamtgewicht der Zubereitung.

18. Verfahren nach einem der Anspruche 16 und 17, dadurch gekennzeichnet, daß der Schritt der Reifung bei einer Temperatur zwischen 15 und 80 °C während einer Zeitdauer von 1 Stunde bis 15 Tagen durch-

EP 0 452 202 B1

geführt wird.

## Claims

1. Aqueous cosmetic or dermopharmaceutical composition characterized by the fact that it contains, in suspension in the continuous phase, hydrated spheroids of a hydrophilic lipid substance, the said hydrated spheroids having a mean particle diameter of between 50 and 10,000 μm.

2. Composition according to Claim 1, characterized by the fact that the external phase is water or a mixture of water and a hydroxylated organic solvent, the said mixture containing at least 50 % water.

3. Composition according to Claim 1, characterized by the fact that the external phase is a gel, the gelling agent being present in a proportion of between 0.02 and 70 % by weight relative to the total weight of the composition.

4. Composition according to Claim 1, characterized by the fact that the external phase is an emulsion of the oil-in-water or water-in-oil type.

5. Composition according to Claim 1, characterized by the fact that the mean particle diameter is preferably between 100 and 5,000 μm.

6. Composition according to any one of Claims 1 to 5, characterized by the fact that the hydrophilic lipid substance is a $C_{12}$-$C_{24}$ fatty alcohol having a melting point of between 20 and 80°C and having a hydroxyl value of between 100 and 300.

7. Composition according to any one of Claims 1 to 5, characterized by the fact that the hydrophilic lipid substance is a partial ester of $C_{12}$-$C_{24}$ fatty acids with polyols or oligomers of polyols, having a melting point of between 20 and 80°C and a hydroxyl value of between 50 and 500 or having a HLB of between 1 and 13.

8. Composition according to any one of Claims 1 to 5, characterized by the fact that the hydrophilic lipid substance is a fat derivative oxyethylenated by means of 2 to 50 moles of ethylene oxide, having a melting point of between 20 and 80°C and having a HLB of between 1 and 13.

9. Composition according to any one of Claims 1 to 5, characterized by the fact that the hydrophilic lipid substance is a product obtained from alcoholysis reaction between natural triglycerides and polyoxyethylene glycols, having a melting point of between 20 and 80°C, a hydroxyl value of between 50 and 500 or a HLB of between 1 and 13.

10. Composition according to any one of Claims 1 to 5, characterized by the fact that the hydrophilic lipid substance is a phospholipid or a sphingolipid or one of their derivatives having a melting point greater than 20°C and having a hydroxyl value of between 50 and 500 or a HLB of between 1 and 13.

11. Composition according to any one of Claims 1 to 4, characterized by the fact that the hydrophilic lipid substance is an amphiphilic silicone having a melting point of between 20 and 80°C and comprising 2 to 50 moles of ethylene oxide per mole of product.

12. Composition according to any one of the preceding claims, characterized by the fact that the hydrated spheroids are charged with at least one fat-soluble or fat-insoluble cosmetic or dermopharmaceutical component.

13. Composition according to Claim 12, characterized by the fact that the hydrated spheroids are charged with a perfume, an essential oil, a pigment, a filler, an abrasive substance, a colorant and/or a cosmetic or dermopharmaceutical active substance.

14. Composition according to either of Claims 12 and 13, characterized by the fact that the hydrated spheroids are charged with at least one cosmetic ingredient in an amount of 0.01 to 70 % and preferably of 1 to 40 % by weight relative to the weight of the spheroids.

15

15. Composition according to any one of the preceding claims, characterized by the fact that the continuous phase contains at least one water-soluble cosmetic or dermopharmaceutical adjuvant chosen from the group consisting of: a colorant, a moisturizing agent, a biological extract, a vitamin and an amino acid.

16. Process for preparing the compositions according to any one of Claims 1 to 15, characterized by the fact that it consists in incorporating into the continuous phase, spheroids of a hydrophilic lipid substance, in solid form, the said spheroids being charged or not charged, so as to form a suspension and in subjecting the composition to a maturation step so as bring about the hydration and swelling of the spheroids.

17. Process according to Claim 16, characterized by the fact that the spheroids are incorporated into the composition in a proportion of between 0.1 and 50 % by weight and preferably between 1.5 and 10 % relative to the total weight of the composition.

18. Process according to either of Claims 16 and 17, characterized by the fact that the maturation step is performed at a temperature of between 15°C and 80°C for a period of one hour to 15 days.